# EUROPEAN PATENT APPLICATION

(11) **EP 3 395 322 A1**
(43) Date of publication of application: **31.10.2018**
(21) Application number: 16877958.5
(22) Date of filing: 06.12.2016
(51) Int. Cl.: A61K 8/29, A61Q 17/04, B82Y 30/00, C01B 33/18

(54) **COMPOSITE PARTICLES IN WHICH TITANIUM DIOXIDE MICROPARTICLES ARE DISPERSED, AND COSMETIC**

(30) Priority: 25.12.2015 JP 2015253568
(71) Applicant: Nippon Sheet Glass Company, Limited, Tokyo 108-6321 (JP)
(72) Inventor: SHIMOKAWA, Kosei, Tokyo 108-6321 (JP); DOSHITA, Kazuhiro, Tokyo 108-6321 (JP); YAMAJI, Koichi, Osaka-shi Osaka 551-0022 (JP); YAMASHITA, Jun, Osaka-shi Osaka 551-0022 (JP)
(74) Representative: Cabinet Beau de Loménie
(86) International application number: PCT/JP2016/005066
(87) International publication number: WO 2017/110053

(57) **Abstract**

The titanium dioxide fine particle-dispersed composite particles (1) of the present invention include silica particles (10) and fine titanium dioxide particles (20). The fine titanium dioxide particles (20) are dispersed inside the silica particles (10). The fine titanium dioxide particles (20) are of the rutile type and have an average primary particle diameter of 2 nm to 8 nm. The titanium dioxide fine particle-dispersed composite particles (1) of the present invention have high transparency to visible light.

## Description

### TECHNICAL FIELD

The present invention relates to titanium dioxide fine particle-dispersed composite particles and a cosmetic containing the titanium dioxide fine particle-dispersed composite particles.

### BACKGROUND ART

Glass flakes inside of which fine titanium dioxide particles are dispersed are conventionally known. Since titanium dioxide has ultraviolet-shielding ability, such glass flakes are contained in, for example, cosmetics.

Patent Literature 1 describes glass flakes inside of which fine metal oxide particles are dispersed. The fine metal oxide particles, which are derived from colloidal particles having a hydroxy group, have a particle diameter of 1 to 300 nm and are dispersed substantially in the form of single particles without aggregating in the glass flakes. As glass flakes whose fine metal oxide particles are fine titanium dioxide particles, Patent Literature 1 describes glass flakes produced using titanium hydroxide oxide (TiO(OH)₂) containing colloid or hydroxide colloid (refer to Examples 1 to 3). In this case, only anatase titanium dioxide is detected in the glass flakes obtained.

Patent Literature 1 also describes glass flakes produced using fine rutile titanium dioxide particles (refer to Comparative Example 1). In the glass flakes, however, ten thousand to several tens of thousands of fine titanium dioxide particles are aggregated to form an aggregate having a diameter of about several microns. Moreover, the glass flakes have a low visible light transmittance.

### CITATION LIST

### Patent Literature

Patent Literature 1: JP 07-315859 A

### SUMMARY OF INVENTION

### Technical Problem

According to the technique described in Patent Literature 1, it is difficult to produce glass flakes having high transparency to visible light using fine rutile titanium dioxide particles.

The present invention therefore aims to provide composite particles having high transparency to visible light and containing fine rutile titanium dioxide particles dispersed in silica particles.

### Solution to Problem

The present invention provides titanium dioxide fine particle-dispersed composite particles including:
silica particles; and
fine rutile titanium dioxide particles dispersed inside the silica particles and having an average primary particle diameter of 2 nm to 8 nm.

The present invention also provides a cosmetic including the titanium dioxide fine particle-dispersed composite particles.

### Advantageous Effects of Invention

Although the fine rutile titanium dioxide particles are dispersed inside the silica particles, the above titanium dioxide fine particle-dispersed composite particles have high transparency to visible light. In the above cosmetic, ultraviolet-shielding ability is exhibited owing to the titanium dioxide fine particle-dispersed composite particles and impairment of the transparency to visible light is prevented by the titanium dioxide fine particle-dispersed composite particles.

### BRIEF DESCRIPTION OF DRAWINGS

FIG. 1 schematically shows a titanium dioxide fine particle-dispersed composite particle according to the present invention.
FIG. 2 is a transmission electron microscope (TEM) image of composite particles according to Example 1.
FIG. 3 is a TEM image of composite particles according to Comparative Example 1.
FIG. 4 is a TEM image of composite particles according to Comparative Example 2.
FIG. 5 is a graph showing a relation between the transmittance of the composite particles according to Example and Comparative Examples and the wavelength of transmitted light.
FIG. 6 is a graph showing transmittance curves of cosmetics according to Example and Comparative Examples.

### DESCRIPTION OF EMBODIMENTS

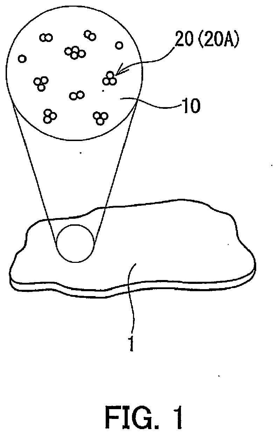

As shown in FIG. 1, a titanium dioxide fine particle-dispersed composite particle 1 includes a silica particle 10 and fine rutile titanium dioxide particles 20. The silica particle 10 is a matrix for binding the fine titanium dioxide particles 20. The fine titanium dioxide particles 20 are dispersed inside the silica particle 10. The fine titanium dioxide particles 20 have an average primary particle diameter of 2 nm to 8 nm. The average primary particle diameter of the fine titanium dioxide particles 20 can be determined by taking images of 1000 or more fine titanium dioxide particles 20 with a TEM, performing image processing on the obtained TEM images of the particles using an image-analyzing particle size distribution analyzer to determine unidirectional particle diameters (distance between two lines being parallel to a specified direction and sandwiching a projection image) of the particles, and calculating the arithmetic mean of the unidirectional particle diameters.

According to conventional techniques, it has been thought that if fine rutile titanium dioxide particles have such a small average primary particle diameter, the dioxide fine particles are aggregated together inside silica particle serving as a matrix and thus formed aggregates larger than 100 nm. The aggregates of fine titanium dioxide particles which are larger than 100 nm are likely to scatter visible light. Therefore, it has been thought difficult to obtain composite particles having silica particles as a matrix and showing a high transmittance to visible light (wavelength: 400 nm to 800 nm) by use of fine rutile titanium dioxide particles having a small primary particle diameter.

In fact, in glass flakes described in Patent Literature 1 produced by use of fine rutile titanium dioxide particles having a primary particle diameter of 20 nm to 50 nm, there are few fine titanium dioxide particles monodispersed in a silica glass matrix, and ten thousand to several tens of thousands of the fine titanium dioxide particles are aggregated to form an aggregate having a diameter of about several microns. A resin film in which the glass flakes are dispersed has a low visible light transmittance at a wavelength ranging from 400 to 800 nm.

The present inventors investigated materials and production conditions of the titanium dioxide fine particle-dispersed composite particles 1 through much trial and error. As a result, the present inventors successfully dispersed fine rutile titanium dioxide particles having an average primary particle diameter of 2 nm to 8 nm in silica particles as a matrix and obtained composite particles having a high visible light transmittance. The present inventors thus completed the present invention.

As shown in FIG. 1, the fine titanium dioxide particles 20 are dispersed inside the silica particle 10 appropriately. For example, the fine titanium dioxide particles 20 form aggregates 20A having a secondary particle diameter of 10 nm to 100 nm inside the silica particles 10. This inhibits the fine titanium dioxide particles 20 from scattering the light passing through the titanium dioxide fine particle-dispersed composite particles 1. Consequently, the titanium dioxide fine particle-dispersed composite particles 1 have a high visible light transmittance. The particle diameter (secondary particle diameter) of the aggregates 20A is desirably 10 nm to 50 nm, and more desirably 10 nm to 20 nm.

In the titanium dioxide fine particle-dispersed composite particles 1, some of the fine titanium dioxide particles 20 may exist as single particles without aggregating. Within a limited region of the titanium dioxide fine particle-dispersed composite particles 1, the fine titanium dioxide particles 20 may form aggregates larger than 100 nm. Desirably, on a number basis, 30% or more of the fine titanium dioxide particles 20 form a plurality of aggregates 20A. This can be confirmed by, for example, TEM observation of the dispersion state of 100 or more fine titanium dioxide particles 20 in the titanium dioxide fine particle-dispersed composite particles 1.

The content of the fine titanium dioxide particles 20 in the titanium dioxide fine particle-dispersed composite particles 1 is, for example, 0.1 mass% to 80 mass%, and desirably 20 mass% to 50 mass%. This makes it easy to appropriately disperse the fine titanium dioxide particles 20 in the titanium dioxide fine particle-dispersed composite particles 1. Therefore, the titanium dioxide fine particle-dispersed composite particles 1 are likely to have a high visible light transmittance and desired ultraviolet-shielding ability.

The titanium dioxide fine particle-dispersed composite particles 1 have a high visible light transmittance. For example, the titanium dioxide fine particle-dispersed composite particles 1 are dispersed in ion-exchange water so that the concentration of the fine titanium dioxide particles will be 0.1 mass%, and the resultant dispersion is subjected to a 5-minute sonication to prepare a specimen. When this specimen is put in a cell having an optical path length of 2 mm and measured with a spectrophotometer, the light transmittance at a wavelength ranging from 400 nm to 700 nm was 80% or higher. When this specimen is put in a cell having an optical path length of 2 mm and measured with a spectrophotometer, the light transmittance at a wavelength of 350 nm was 50% or lower. As demonstrated above, the titanium dioxide fine particle-dispersed composite particles 1 have ultraviolet-shielding ability and also have a high visible light transmittance.

The shape of the titanium dioxide fine particle-dispersed composite particles 1 is, for example, but not particularly limited to, flaky as shown in FIG. 1. The term "flaky" as used herein means the shape of a plate whose principal surfaces can be regarded as flat or curved surfaces and in which the ratio of the average diameter of each principal surface to the thickness is 3 or more. The average diameter of the titanium dioxide fine particle-dispersed composite particles 1 is the diameter of a circle having an area equal to the area of the principal surface of the titanium dioxide fine particle-dispersed composite particles 1. In the case where the titanium dioxide fine particle-dispersed composite particles 1 are flaky, the thickness thereof is, for example, 0.1 µm to 5 µm, and desirably 0.1 µm to 2 µm. The average particle diameter of the titanium dioxide fine particle-dispersed composite particles 1 is, for example, 1 µm to 80 µm, and desirably 3 µm to 40 µm. The average particle diameter of the titanium dioxide fine particle-dispersed composite particles 1 means the median particle diameter (D50) in the volume-based particle size distribution measured using a laser diffraction granulometer.

A cosmetic including the titanium dioxide fine particle-dispersed composite particles 1 can be provided using the titanium dioxide fine particle-dispersed composite particles 1. By virtue of including the titanium dioxide fine particle-dispersed composite particles 1, this cosmetic has ultraviolet-shielding ability. Furthermore, since the titanium dioxide fine particle-dispersed composite particles 1 have a high visible light transmittance, impairment of the transparency to visible light is prevented by the titanium dioxide fine particle-dispersed composite particles 1. The content of the titanium dioxide fine particle-dispersed composite particles 1 in the cosmetic is, for example, but not particularly limited to, 0.1 mass% to 50 mass%. This makes it likely that the cosmetic has desired ultraviolet-shielding ability. This can also inhibit excessive reduction of the fluidity of the cosmetic.

Hereinafter, an example of a method for producing the titanium dioxide fine particle-dispersed composite particles 1 will be described. A silica sol containing colloidal silica particles as a dispersoid and water as a dispersion medium is prepared. The pH of the silica sol is, for example, 7 or higher. The particle diameter of the colloidal silica particles in the silica sol is, for example, 7 nm to 20 nm. A titania sol containing colloidal particles of rutile titanium dioxide as a dispersoid and water as a dispersion medium is also prepared. The pH of the titania sol is, for example, 6 to 7. The particle diameter of the colloidal particles of titanium dioxide in the titania sol is, for example, 2 nm to 8 nm. The silica sol and titania sol are mixed to obtain a mixed sol. The mixing ratio of the silica sol and titania sol is not particularly limited. For example, the silica sol and titania sol are mixed so that the mass of the solids of the titania sol in the mixed sol will be 0.1% to 60% of the mass of the total solids in the mixed sol.

Next, the mixed sol is introduced into a liquid containing an organic solvent. As the organic solvent, for example, a protic solvent having a relative permittivity of 30 or less at 20°C and being miscible with water or an aprotic solvent having a relative permittivity of 40 or less at 20°C and being miscible with water can be used. The mixed sol is introduced, for example, dropwise into the liquid containing the predetermined organic solvent. At this time, it is desirable that the mixed sol be introduced into the liquid containing the predetermined organic solvent under stirring. Flaky aggregates of the colloidal silica particles are thus yielded in the liquid containing the predetermined organic solvent. At this time, the fine rutile titanium dioxide particles are included and dispersed inside the aggregates. In this manner, a slurry in which the flaky aggregates of the colloidal particles are formed can be obtained.

Next, the solvent is removed from the slurry in which the flaky aggregates of the colloidal particles are formed. The removal of the solvent can be performed, for example, by vacuum-drying the slurry in a vacuum drying oven. The drying also turns the aggregates to a gel. This gelation increases the binding force between the colloidal silica particles composing the yielded flaky aggregates. The gel aggregates are baked as needed to remove an unnecessary organic substance. The baking is carried out, for example, at a temperature ranging from 400°C to 1100°C, and desirably at a temperature ranging from 500°C to 900°C. The titanium dioxide fine particle-dispersed composite particles 1 being flaky can thus be obtained.

### EXAMPLES

The present invention will be described in more detail by way of examples. It should be noted that the present invention is not limited to examples given below.

### <Evaluation of light transmittance of composite particles>

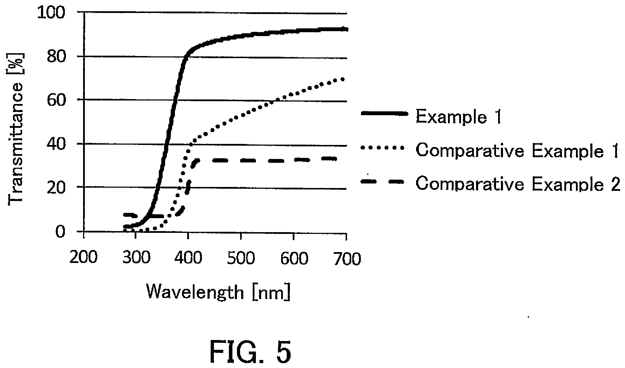

First, a method for evaluating the light transmittance of composite particles according to Example 1, Comparative Example 1, and Comparative Example 2 is described. The composite particles according to Example 1, Comparative Example 1, and Comparative Example 2 were individually dispersed in ion-exchange water so that the concentration of the fine titanium dioxide particles would be 0.1 mass%, and the resultant dispersions were subjected to a 5-minute sonication to prepare specimens. These specimens were each put in a cell having an optical path length of 2 mm to measure the light transmittance at a wavelength ranging from 280 nm to 700 nm using a spectrophotometer (manufactured by Shimadzu Corporation, product name: UV-3600). The measurement results are shown in FIG. 5.

### <Evaluation of transparency of cosmetic>

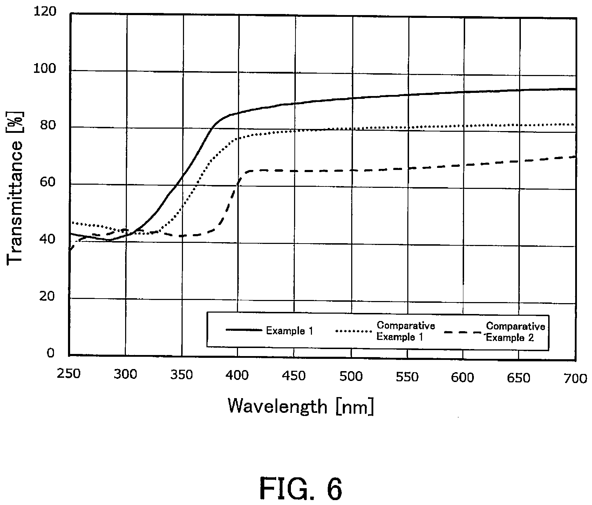

Cosmetics according to Example 1, Comparative Example 1, and Comparative Example 2 were each used to form a uniform coating having a film thickness of about 10 µm on a polypropylene film. A transmittance curve of each obtained coating was determined using a spectrophotometer (manufactured by Hitachi High-Technologies Corporation, product name: U-4100). The measurement results are shown in FIG. 6. From the transmittance curve of each coating, an integral of the transmittance over wavelengths in the visible light range (400 nm to 700 nm) was also determined. The results are shown in Table 2.

### <Evaluation of ultraviolet-shielding property of cosmetic>

An amount of 2 mg/cm² of each of the cosmetics according to Example 1, Comparative Example 1, and Comparative Example 2 was applied to a polymethyl methacrylate (PMMA) plate to form a coating, which was measured for a sun protection factor (SPF) using a SPF analyzer (manufactured by Labsphere, Inc., product name: UV-2000S). In this manner, the ultraviolet-shielding property was measured for the cosmetics according to Example 1, Comparative Example 1, and Comparative Example 2. The measurement results are shown in Table 2.

### <Example 1>

### (Production of composite particles)

A mixed sol was obtained by mixing an alkaline silica sol (manufactured by Nippon Chemical Industrial Co., Ltd., product name: SILICADOL 30S) and titania sol (manufactured by Tayca Corporation, product name: TS-226) in such a manner that the mass of the solids of the titania sol was about 25% of the mass of the total solids. SILICADOL 30S is a colloidal silica containing water as a dispersion medium and having a pH of 9.0 to 10.5, and the particle diameter of the colloidal particles contained therein is 7 nm to 10 nm. TS-226 is a sol containing fine rutile titanium dioxide particles as a dispersoid and water as a dispersion medium. The content of the fine titanium dioxide particles in TS-226 is 17.9%. The pH of TS-226 is 6 to 7, and the particle diameter of the fine titanium dioxide particles, which are colloidal particles in TS-226, is 2 to 8 nm.

Next, 50 ml of 2-propanol was put in a beaker, and a total amount of 1 g of the mixed sol was added in drops of 0.01 g to the 2-propanol. During the dropping of the mixed sol, the 2-propanol was stirred with a magnetic stirrer at a rotational speed of 800 ppm. In this manner, a slurry in which flaky aggregates of the colloidal particles were formed was obtained. This slurry was put in a vacuum drying oven set at 120°C to remove the solvent, and thus dry aggregates were obtained. This was followed by baking the dried aggregates at 600°C for 5 hours. Composite particles according to Example 1 were obtained in this manner.

### (Preparation of cosmetic)

A mixture containing the components shown in Table 1 was heated to dissolve the components, and thus a phase A was obtained. A mixture containing the components shown in Table 1 was heated to dissolve the components, and thus a phase B was obtained. The composite particles according to Example 1 were added to the phase B in an amount shown in Table 1 and uniformly dispersed in the phase B using a dispersion mixer. Subsequently, the phase A was added to the dispersion in which the composite particles according to Example 1 were uniformly dispersed in the phase B. The resultant mixture was emulsified with a homomixer under heating, and then cooled down to 30°C to obtain a cosmetic according to Example 1. The cosmetic according to Example 1 was an oil-in-water cosmetic.

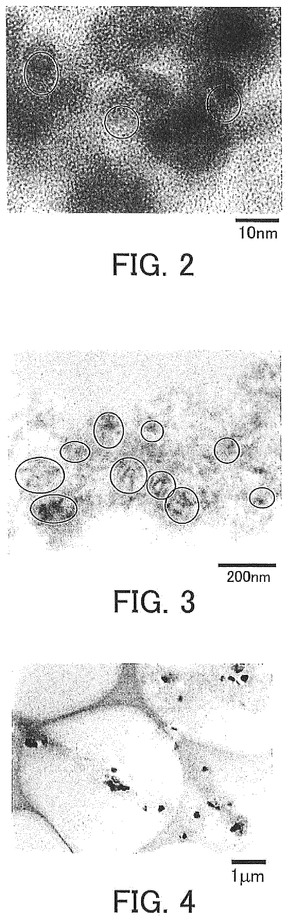

As shown in FIG. 2, the composite particles according to Example 1 were composite particles in which fine rutile titanium dioxide particles having an average primary particle diameter of 6 nm were dispersed inside the silica particles. Fine titanium dioxide particles are present at the circled spots in FIG. 2. The average particle diameter of the composite particles according to Example 1 was about 20 µm, and the average thickness thereof was about 1 µm. The content of the fine titanium dioxide particles in the composite particles according to Example 1 was about 20 mass%. From the TEM image of the composite particles according to Example 1, it was confirmed that, inside the silica particles, most of the fine titanium dioxide particles formed aggregates having a secondary particle diameter of 10 nm to 20 nm.

### <Comparative Example 1>

An amount of 500 g of fine titanium dioxide particles (manufactured by Tayca Corporation, product name: MT-100 AQ, average primary particle diameter: 15 nm) and 42 g of an ammonium polyacrylate surfactant (manufactured by Cognis Corporation, product name: HYDROPALAT 5050) were added to 1125 g of pure water. The mixture was circulated and stirred (stirring speed: a circumferential speed of 8 m/sec, flow rate: 1 L/min) for 2 hours together with 4 kg of zirconia beads of 0.65 mm diameter using a horizontal continuous wet-type bead mill (manufactured by Shinmaru Enterprises Corporation, product name: DYNO-MILL KDL-PILOT A). Thus, a dispersion A1 of the fine titania dioxide particles was obtained.

Next, 4.29 parts by mass of the dispersion A1 of the fine titanium dioxide particles was mixed with 10.0 parts by mass of a silica sol (manufactured by Nippon Chemical Industrial Co., Ltd., product name: SILICADOL 30S) to obtain a sol solution A2. The content of the fine titanium dioxide particles in the solids of the sol solution A2 was 30 mass%. Subsequently, composite particles according to Comparative Example 1 were obtained in the same manner as in Example 1, except for using the sol solution A2 instead of the mixed sol.

A cosmetic according to Comparative Example 1 was obtained in the same manner as in Example 1, except for using the composite particles according to Comparative Example 1 instead of the composite particles according to Example 1. The cosmetic according to Comparative Example 1 was an oil-in-water cosmetic.

The average particle diameter of the composite particles according to Comparative Example 1 was about 17 µm, and the average thickness thereof was about 1 µm. The content of the fine titanium dioxide particles in the composite particles according to Comparative Example 1 was 30 mass%. From the TEM image of the composite particles according to Comparative Example 1, it was confirmed that, inside the silica particles, many of the fine titanium dioxide particles formed aggregates having a secondary particle diameter of 100 nm to 200 nm.

### <Comparative Example 2>

An amount of 500 g of fine titanium dioxide particles (manufactured by ISHIHARA SANGYO KAISHA, LTD., product name: CR-50, average primary particle diameter: 250 nm) and 42 g of an ammonium polyacrylate surfactant (manufactured by Cognis Corporation, product name: HYDROPALAT 5050) were added to 1125 g of pure water. The mixture was circulated and stirred (stirring speed: a circumferential speed of 8 m/sec, flow rate: 1 L/min) for 2 hours together with 4 kg of zirconia beads of 0.65 mm diameter using a horizontal continuous wet-type bead mill (manufactured by Shinmaru Enterprises Corporation, product name: DYNO-MILL KDL-PILOT A). Thus, a dispersion B1 of the fine titania dioxide particles was obtained.

Next, 4.29 parts by mass of the dispersion B1 of the fine titanium dioxide particles was mixed with 10.0 parts by mass of a silica sol (manufactured by Nippon Chemical Industrial Co., Ltd., product name: SILICADOL 30S) to obtain a sol solution B2. The content of the fine titanium dioxide particles in the solids of the sol solution B2 was 30 mass%. Subsequently, composite particles according to Comparative Example 2 were obtained in the same manner as in Example 1, except for using the sol solution B2 instead of the mixed sol.

A cosmetic according to Comparative Example 2 was obtained in the same manner as in Example 1, except for using the composite particles according to Comparative Example 2 instead of the composite particles according to Example 1. The cosmetic according to Comparative Example 2 was an oil-in-water cosmetic.

The average particle diameter of the composite particles according to Composite Example 2 was about 17 µm, and the average thickness thereof was about 1 µm. The content of the fine titanium dioxide particles in the composite particles according to Comparative Example 2 was 30 mass%. From the TEM image of the composite particles according to Comparative Example 2, it was confirmed that, inside the silica particles, many of the fine titanium dioxide particles formed aggregates having a secondary particle diameter of 300 nm to 500 nm.

As shown in FIG. 5, it was indicated that the composite particles according to Example 1 had a high visible light transmittance. It was also indicated that the composite particles according to Example 1 had ultraviolet-shielding ability. Both the composite particles according to Comparative Examples 1 and 2 could hardly be said to have a high visible light transmittance.

As shown in FIG. 6 and Table 2, it was indicated that the cosmetic according to Example 1 had a high visible light transmittance. The cosmetics according to Comparative Examples 1 and 2 could hardly be said to have a visible light transmittance as high as that of the cosmetic according to Example 1.

As shown in Table 2, the cosmetic according to Example 1 showed a high SPF. Both of the cosmetics according to Comparative Examples 1 and 2 could hardly be said to show SPFs as high as that of the cosmetic according to Example 1.

**[Table 1]**

| Component | | Content [mass%] |
|---|---|---|
| Phase A | Mineral oil | 9.0 |
| | Glyceryl caprylate/caprate | 8.0 |
| | Vaseline | 2.0 |
| | Cetyl palmitate | 4.0 |
| | Glyceryl stearate-6 | 0.9 |
| | Glyceryl stearate SE | 2.5 |
| | Behenyl alcohol | 0.9 |
| | Propylparaben | 0.1 |
| Phase B | Water | Adjusted according to content of other components |
| | 1,-butylene glycol (BG) | 6.5 |
| | Methylparaben | 0.2 |
| | Glyceryl stearate-10 | 0.8 |
| Composite particles according to Example 1 | | 13.3 |
| Total | | 100.0 |

**[Table 2]**

| | Integral transmittance from 400 nm to 700 nm [%• nm] | SPF |
|---|---|---|
| Example 1 | 27,620 | 9 |
| Example 2 | 25,968 | 7 |
| Example 3 | 22,757 | 2 |

## Claims

1. Titanium dioxide fine particle-dispersed composite particles comprising:
silica particles; and
fine rutile titanium dioxide particles dispersed inside the silica particles and having an average primary particle diameter of 2 nm to 8 nm.

2. The titanium dioxide fine particle-dispersed composite particles according to claim 1, wherein the fine titanium dioxide particles form an aggregate having a secondary particle diameter of 10 nm to 100 nm inside the silica particles.

3. The titanium dioxide fine particle-dispersed composite particles according to claim 1 or 2, being flaky.

4. A cosmetic comprising the titanium dioxide fine particle-dispersed composite particles according to any one of claims 1 to 3.
